# EUROPEAN PATENT SPECIFICATION

(11) **EP 1 224 466 B1**
(45) Date of publication and mention of the grant of the patent: **03.01.2007**
(21) Application number: 00968823.5
(22) Date of filing: 06.10.2000
(51) Int. Cl.: G01N 33/574

(54) **PROSTATE CANCER MARKER PROTEINS**
MARKERPROTEINE FÜR PROSTATAKREBS
PROTEINES MARQUEUR DU CANCER DE LA PROSTATE

(30) Priority: 07.10.1999 US 158422 P
(43) Date of publication of application: 24.07.2002
(73) Proprietor: Ciphergen Biosystems, Inc., Fremont, CA 94555 (US); Eastern Virginia Medical School, Norfolk, VA 23507 (US)
(72) Inventor: YIP, Tai-Tung, Cupertino, CA 95014 (US); YIP, Christine, Cupertino, CA 95014 (US); WRIGHT, George, L., Jr., Virginia Beach, VA 23455 (US)
(74) Representative: Naylor, Kathryn May
(86) International application number: PCT/US2000/027682
(87) International publication number: WO 2001/025791

(56) References cited:
- WO-A-96/00503
- WO-A-97/12624
- WO-A-98/40738
- WO-A-99/37811
- WO-A-99/61471
- WO-A1-96/40754
- WO-A2-99/12040
- WO-A2-99/27366
- MALM JOHAN ET AL: "Isolation and characterization of the major gel proteins in human semen, semenogelin I and semenogelin II." EUROPEAN JOURNAL OF BIOCHEMISTRY, vol. 238, no. 1, 1996, pages 48-53, XP000997222 ISSN: 0014-2956
- DUBE JEAN Y: "Can prostatic kallikrein kH2 favor prostatic cancer progression?" M-S (MEDECINE SCIENCES), vol. 14, no. 1, January 1998 (1998-01), pages 111-113, XP000997204 ISSN: 0767-0974
- DENMEADE S R ET AL: "SPECIFIC AND EFFICIENT PEPTIDE SUBSTRATES FOR ASSAYING THE PROTEOLYTIC ACTIVITY OF PROSTATE-SPECIFIC ANTIGEN" CANCER RESEARCH,AMERICAN ASSOCIATION FOR CANCER RESEARCH, BALTIMORE, MD,US, vol. 57, 1 November 1997 (1997-11-01), pages 4924-4930, XP002070515 ISSN: 0008-5472
- GROVER P.K.; RESNICK M.I.: 'ANALYSIS OF PROSTATIC FLUID: EVIDENCE FOR THE PRESENCE OF A PROSPECTIVE MARKER FOR PROSTATIC CANCER' PROSTATE vol. 26, no. 1, 1995, WILEY-LISS, NEW YORK, NY, US, pages 12 - 18, XP008045235
- GROVER P.K.; RESNICK M.I.: 'HIGH RESOLUTION TWO-DIMENSIONAL ELECTROPHORETIC ANALYSIS OF URINARY PROTEINS OF PATIENTS WITH PROSTATIC CANCER' ELECTROPHORESIS vol. 18, no. 5, 1997, WILEY-VCH VERLAG, WEINHEIM, DE, pages 814 - 818, XP008045238
- FERNANDEZ-POL J.A. ET AL: 'EXPRESSION OF METALLOPANSTIMULIN AND ONCOGENESIS IN HUMAN PROSTATIC CARCINOMA' ANTICANCER RESEARCH vol. 17, no. 3A, 1997, HELENIC ANTICANCER INSTITUTE, ATHENS, GR, pages 1519 - 1530, XP008045237

### SEQUENCE LISTING

<110> Yip, Tai-Tung
   Yip, Christine
   Wright Jr., George L.
   Ciphergen Biosystems, Inc.
   Eastern Virginia Medical School
<120> Prostate Cancer Marker Proteins
<130> 016866-003810PC
<140> WO PCT/US00/27682
   <141> 2000-10-06
<150> US 60/158,422
   <151> 1999-10-07
<160> 1
<170> PatentIn Ver. 2.1
<210> 1
   <211> 52
   <212> PRT
   <213> Homo sapiens
<220>
   <223> seminal basic protein, peptide fragment amino acid positions 85-136 of semenogelin I
<400> 1
   His Asn Lys Gln Glu Gly Arg Asp His Asp Lys Ser Lys Gly His Phe
    1               5                  10                  15
   His Arg Val Val Ile His His Lys Gly Gly Lys Ala His Arg Gly Thr
               20                  25                  30
   Gln Asn Pro Ser Gln Asp Gln Gly Asn Ser Pro Ser Gly Lys Gly Ile
           35                  40                  45
   Ser Ser Gln Tyr
       50

## Description

### BACKGROUND OF THE INVENTION

Prostate cancer is the most common form of cancer in males. It typically afflicts aging males, but it can afflict males of all ages. A significant number of males die from prostate cancer every year, and it is the second leading cause of cancer deaths in men. Early diagnosis of prostate cancer in patients reduces the likelihood of death.

Conventionally, prostate cancer is diagnosed using prostate specific antigen (PSA) as a marker. PSA is a 33 kDa glycoprotein synthesized and secreted mainly by the prostate gland. PSA is also a protease with serine protease activity and cleaves proteins at the leucine or tyrosine residue *(see, e.g.,* Christensson *et al., Eur. J. Biochem.* 194:755-763 (1990)). Its natural substrates are semenogelin I, semenogelin II and fibronectin in the seminal plasma *(see, e.g.,* Lilja, *J. Clin. Invest.* 76:1899 (1985); Lilja *et al., J. Clin. Invest.* 80:281 (1987); McGee and Herr, *Biol. Reprod.* 39:499 (1988)). These proteins are mainly responsible for the immediate gel formation of freshly ejaculated semen. The PSA-mediated proteolysis of these gel-forming proteins results in the liquefaction of semen and the release of progressively motile spermatozoa.

PSA leaks into the blood stream, and measurements of the concentration of PSA in the blood serum have now found widespread use in detecting prostate cancer in people. In a typical procedure, an immunoassay is performed to measure the level of PSA in a patient's serum. Blood serum levels of PSA in normal males are typically very low (*e.g*., less than 1 ng/ml). In general, PSA levels above 4 ng/ml are suggestive of prostate cancer while levels above 10 ng/ml are highly suggestive of prostate cancer.

Although such procedures are effective in some instances, conventional methods for detecting prostrate cancer have limitations. For example, if the cancer is in its early stages, some prostate cancer patients exhibit normal PSA levels at the time of diagnosis. Since conventional PSA tests detect abnormal levels of PSA, conventional PSA tests may not be able to detect the presence of prostate cancer if it is in its early stages. The inability of conventional PSA tests to diagnose the presence of prostate cancer in some instances (*e.g.,* in the early stages of the disease) can be detrimental to the patient. Moreover, many individuals with elevated levels of PSA in the blood serum may not have prostate cancer, but may instead have benign prostate hyperplasia (BPH) (*i.e.,* a benign tumor). In order to determine if a person has prostate cancer, rather than BPH, additional immunoassays using other antibodies and/or biopsies of the prostate tissue are performed. These additional tests are time consuming for both patients and their care providers.

Grover and Resnick *et al* (The Prostate, 26:12-18 (1995)) describe a 22kDa protein as present in patients with prostate carcinoma. Fernandez-Pol *et al* (Anticancer Research 17:1519-1530 (1997)) describe the presence of metalloproteinstimulin in the blood of prostate cancer patients. WO99/12040 relates to a method of determining the identity of polypeptides using mass spectroscopy.

Accordingly, it would be desirable to provide for quick and accurate methods and kits for determining if a person has prostate cancer.

### SUMMARY OF THE INVENTION

The present invention provides, for the first time, sensitive and quick methods that can be used as an aid for diagnosis of prostate cancer by measuring markers that are differentially present in samples of a prostate cancer patient and a subject who does not have prostate cancer (BPH patients). Thus, in a first aspect, the present invention provides a method for aiding a prostate cancer diagnosis, the method comprising:
i. determining by mass spectroscopy a test amount of a plurality of protein markers in a sample from a subject, the protein markers having apparent molecular weights of less than 10,000 Da;
ii. comparing the test amount of the plurality of protein markers having apparent molecular weight of less than 10,000 Da with an amount of a plurality of protein markers having an apparent molecular weight of less than 10,000 Da from a control sample where the control sample originates from benign prostate hyperplasia; and
iii. determining whether the test amount is a diagnostic amount consistent with a diagnosis of prostate cancer versus benign hyperplasia.

By monitoring the amount of these markers, the methods and kits disclosed herein can determine the subject's pathological status using minute quantities of crude samples. In particular, seminal basic protein is a positive marker differentiating prostate cancer and BPH.

Thus, the invention relates to methods for aiding a prostate cancer diagnosis, which comprise determining a test amount of a marker in a sample from a subject and determining whether the test amount is a diagnostic amount consistent with a diagnosis of prostate cancer. A test amount of a plurality of markers are determined in the present invention.

The markers can have any suitable characteristics, including apparent molecular weight of less than 10,000 Da. Also disclosed herein are markers having an apparent molecular weight of less than 27,000 Da, less than about 15,000 Da. In one embodiment, the markers are present at an elevated level in samples of prostate cancer patients compared to samples of BPH patients. For example, these markers include polypeptides having an apparent molecular weight of about 2776 Da, 4423 Da, 4480 Da, 5753 Da, 6098 Da, 6270 Da, 6998 Da, 7843 Da, 8030 Da, 8240 Da, or 8714 Da. In yet another embodiment, the markers are present at an elevated level in samples of BPH patients compared to samples of prostate cancer patients. For examples, these markers include polypeptides having an apparent molecular weight of about 2276 Da, 2530 Da, 2095 Da, 3030 Da, 3038 Da, 3224 Da, 3600 Da, 3835 Da, 3915 Da, 3933 Da, or 4175 Da. In one preferred embodiment, the marker being detected includes seminal basic protein which has an apparent molecular weight of about 5753 Da.

In another embodiment, the markers include a cleaved product generated by PSA-mediated proteolysis. For example, the cleaved product is generated by PSA-mediated proteolysis of semenogelin I. In a preferred embodiment, the cleaved product is seminal basic protein.

In yet another embodiment, a sample being tested is taken from a subject's blood, serum, urine, semen, seminal fluid, seminal plasma, or tissue extracts. Preferably, the sample is seminal plasma.

Also disclosed herein are methods for diagnosis comprising determining a test amount of a marker in a sample using immunoassay or gas phase ion spectrometry. Preferably, laser desorption mass spectrometry is used.

The invention relates to a method for detecting a marker, the method comprising contacting a sample from a subject with a substrate comprising an adsorbent thereon under conditions to allow binding between a marker and the adsorbent, wherein the marker is a polypeptide which is differentially present in samples of a prostate cancer and a subject who does not have prostate cancer (*e.g.*, BPH patients), and detecting the marker bound to the adsorbent by gas phase ion spectrometry. Also disclosed the markers having an apparent molecular weight of less than 27,000 Da.

In another embodiment, the adsorbent comprises a hydrophobic group and an anionic group. In another embodiment, the substrate comprises a polystyrene latex bead functionalized with a sulfonate group as an adsorbent. In yet another embodiment, the adsorbent comprises a hydrophobic group. In yet another embodiment, the adsorbent comprises a metal chelating group complexed with a metal ion. In yet another embodiment, the adsorbent comprises an antibody that specifically binds to a marker. In yet another embodiment, the adsorbent comprises an antibody that specifically binds to seminal basic protein.

In yet another embodiment, prior to detecting the marker unbound materials on the substrate is removed with a washing solution. Preferably, the washing solution is an aqueous solution, such as a HEPES buffer, a phosphate buffered saline, or water.

In yet another embodiment, the markers include a cleaved product generated by PSA-mediated proteolysis of a protein substrate, such as semenogelin I. In yet another embodiment, the cleaved product generated by PSA-mediated proteolysis is seminal basic protein.

In yet another embodiment, the method comprises determining the test amount of the markers bound on the probe substrate, and determining whether the test amount is a diagnostic amount consistent with a diagnosis of prostate cancer.

Also disclosed herein is a method for detecting a marker in a sample, the method comprising: providing an antibody that specifically binds to the marker, wherein the marker is a polypeptide which is differentially present in samples of a prostate cancer patient and a subject who does not have prostate cancer (*e.g.,* a BPH patient), and contacting the sample with the antibody, and detecting the presence of a complex of the antibody bound to the marker. In such disclosure, the marker may have an apparent molecular weight of less than 27,000 Da.

As disclosed herein the marker being detected may be a cleaved product generated by PSA-mediated proteolysis of a protein substrate, such as semenogelin I, or is seminal basic protein. The method may further comprise determining the test amount of the markers bound on the probe substrate, and determining whether the test amount is a diagnostic amount consistent with a diagnosis of prostate cancer.

Also disclosed is a kit for aiding a diagnosis of prostate cancer, wherein the kit comprises a substrate comprising an adsorbent thereon, wherein the adsorbent is suitable for binding a marker and a washing solution or instructions for making a washing solution, wherein the combination of the adsorbent and the washing solution allows detection of the marker using gas phase ion spectrometry. The kit is capable of allowing determination of a test amount of a marker, wherein the marker is a polypeptide which is differentially present in samples of a prostate cancer patient and a subject who does not have prostate cancer (*e.g.,* a BPH patient). Such kit can detect a marker which has an apparent molecular weight of less than 10,000 Da.

The substrate in the kit may be in the form of a probe which is removably insertable into a gas phase ion spectrometer. The kit may further comprise another substrate which can be used together with the substrata comprising the adsorbent to form a probe which is removably insertable into a gas phase ion spectrometer.

The kit may further comprise instructions for suitable operational parameters.

The substrate may comprise a hydrophobic group and an anionic group as an adsorbent. In yet another embodiment, the substrate comprises a hydrophobic group as an adsorbent. In yet another embodiment, the substrate comprises a metal chelating group. In yet another embodiment, the substrate comprises a metal chelating group complexed with a metal ion as an adsorbent. In yet another embodiment, the substrate comprises an antibody that specifically binds to a marker, preferably seminal basic protein, as an adsorbent. In yet another embodiment, the washing solution is an aqueous solution.

The kit may comprise an antibody that specifically binds to the marker, and a detection reagent. Optionally, the antibody can be immobilized on a solid support.

The kits can further comprise a standard indicating a diagnostic amount of the marker.

While the absolute identity of many markers is not yet known, such knowledge is not necessary to measure them in a patient sample, because they are sufficiently characterized by, *e.g.,* mass and by affinity characteristics. It is noted that molecular weight and binding properties are characteristic properties of these markers and not limitations on means of detection or isolation. Furthermore, using the methods described herein or other methods known in the art, the absolute identity of the markers can be determined.

These and other aspects of the present invention will become apparent upon reference to the following detailed description and attached drawings.

### BRIEF DESCRIPTION OF THE DRAWINGS

Figure 1 illustrates the amino acid sequence of seminal basic protein (SEQ ID NO:1).
Figure 2 illustrates a probe comprising spots of adsorbents on the probe surface.
Figure 3 illustrates an example of how a test sample is compared to a control as part of prostate cancer diagnosis.
Figure 4 illustrates proteins in samples of a prostate cancer patient and a benign prostate hyperplasia patient that are bound on Ni(II) ProteinChip^{®} array. Figure 4 also illustrates a difference map obtained by subtracting the data obtained from the sample of the benign prostate hyperplasia patient from the data obtained from the sample of the prostate cancer patient.
Figure 5 illustrates proteins in samples of a prostate cancer patient and a benign prostate hyperplasia patient that are bound on H4 ProteinChip^{®} array. Figure 5 also illustrates a difference map obtained by subtracting the data obtained from the sample of the benign prostate hyperplasia patient from the data obtained from the sample of the prostate cancer patient.
Figure 6 illustrates proteins in samples of a prostate cancer patient and a benign prostate hyperplasia patient that are bound on SCX1 ProteinChip^{®} array.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton *et al., Dictionary of Microbiology and Molecular Biology* (2nd ed. 1994); *The Cambridge Dictionary of Science and Technology* (Walker ed., 1988); *The Glossary of Genetics,* 5th Ed., R. Rieger *et al.* (eds.), Springer Verlag (1991); and Hale & Marham, *The Harper Collins Dictionary of Biology* (1991). As used herein, the following terms have the meanings ascribed to them unless specified otherwise.

"Marker" in the context of the present invention refers to a polypeptide which is differentially present in a sample taken from patients having prostate cancer as compared to a comparable sample taken from subjects who do not have prostate cancer, namely, in the context of the invention, benign prostate hyperplasia patients. For example, a marker can be a polypeptide (having a particular apparent molecular weight) which is present at an elevated level in samples of prostate cancer patients compared to samples of BPH patients. In another examples, a marker can be a polypeptide (having a particular apparent molecular weight) which is present at an elevated level in samples of BPH patients compared to samples of prostate cancer patients.

A protein marker is resolved with confidence of about 0.5% variation by gas phase ion spectrometry. Thus, the term "about" in the context of a molecular weight of a marker as measured by mass spectrometry refers to 0.5% variation of the marker molecular weight. For example, the marker with an apparent molecular weight of "about 2776 Da" as measured by mass spectrometry has the apparent molecular weight range of 2776 ± 14 Da; the marker with an apparent molecular weight of "about 4423 Da" as measured by mass spectrometry has the apparent molecular weight range of 4423 ± 22 Da; and so on.

The phrase "differentially present" refers to differences in the quantity and/or frequency of a polypeptide (of a particular apparent molecular weight) present in a sample taken from patients having prostate cancer as compared to a comparable sample taken from patients who do not have prostate cancer (having benign prostate hyperplasia). For example, a marker can be a polypeptide which is present at an elevated level or at a decreased level in samples of prostate cancer patients compared to samples of subjects who have benign prostate hyperplasia. Alternatively, a marker can be a polypeptide which is detected at a higher frequency or at a lower frequency in samples of prostate cancer patients compared to samples of subjects who have benign prostate hyperplasia. A marker can be differentially present in terms of quantity, frequency or both.

A polypeptide is differentially present between the two sets of samples if the frequency of detecting the polypeptide in the prostate cancer patients' samples is statistically significantly higher or lower than in the control samples. For example, two sets of data can be compared using student's t-test, and P<0.05 can be considered statistically significant. In another example, a polypeptide is differentially present between the two sets of samples if it is detected at least about 120%, at least about 130%, at least about 150%, at least about 180%, at least about 200%, at least about 300%, at least about 500%, at least about 700%, at least about 900%, or at least about 1000% more frequently or less frequently observed in one set of samples than the other set of samples.

Alternatively or additionally, a polypeptide is differentially present between the two samples if the amount of the polypeptide in one sample is statistically significantly different from the amount of the polypeptide in the other sample. For example, a polypeptide is differentially present between the two samples if it is present in one sample at least about 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other.

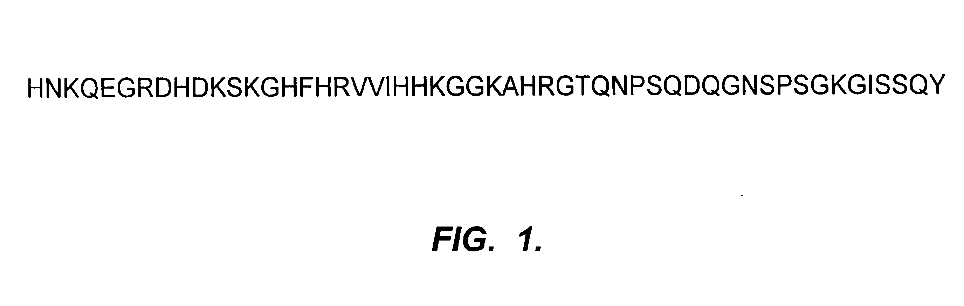

"Seminal basic protein" refers to a peptide fragment generated by prostate-specific antigen-mediated proteolysis of a major protein in seminal plasma, semenogelin I. Seminal basic protein has an apparent molecular weight of about 5753 Da and about 52 amino acid residues (peptide fragment 85-136 of semenogelin I). "Seminal basic protein" can have an amino acid sequence of SEQ ID NO:1 as shown in Figure 1 or a truncated version thereof, and can also include other seminal basic proteins that are polymorphic variants, alleles, mutants, and interspecies homologs of seminal basic protein having an amino acid sequence of SEQ ID NO:1 or truncated versions thereof. *See, also,* Lilja and Jeppsson, *Febs Lett.* 182:181-184 (1985).

"Cleaved product" in the context of the present invention refers to a product that is generated by PSA-mediated proteolysis of a protein substrate, such as semenogelin I.

"Prostate specific antigen-mediated proteolysis" or "PSA-mediated proteolysis" refers to serine protease enzyme activity of prostate specific antigen (PSA).

"Diagnostic" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

A "test amount" of a marker refers to an amount of a marker present in a sample being tested. A test amount can be either in absolute amount (*e.g.,* µg/ml) or a relative amount (*e.g*., relative intensity of signals).

A "diagnostic amount" of a marker in the context of the present invention refers to an amount of a marker in a subject's sample that is consistent with a diagnosis of prostate cancer. A diagnostic amount can be either in absolute amount (*e.g.,* µg/ml) or a relative amount (*e.g.,* relative intensity of signals).

A "control amount" of a marker can be any amount or a range of amount which is to be compared against a test amount of a marker. For example, a control amount of a marker can be the amount of a marker (*e.g*., seminal basic protein) in a prostate cancer patient, a BPH patient or a person without prostate cancer or BPH. A control amount can be either in absolute amount (*e.g.,* µg/ml) or a relative amount (*e.g.,* relative intensity of signals). In the context of the invention the control sample originates from patients having benign prostate hypoplasia.

"Probe" refers to a device that is removably insertable into a gas phase spectrometer and comprises a substrate having a surface for presenting a marker for detection. A probe can comprise a single substrate or a plurality of substrates. Terms such as ProteinChip^{®}, ProteinChip^{®} array, or chip are also used herein to refer to a probe.

"Substrate" or "probe substrate" refers to a solid phase onto which an adsorbent can be provided (*e.g.*, by attachment, deposition, *etc.*)

"Adsorbent" refers to any material capable of adsorbing a marker. The term "adsorbent" is used herein to refer both to a single material ("monoplex adsorbent") (*e.g*., a compound or functional group) to which the marker is exposed, and to a plurality of different materials ("multiplex adsorbent") to which the marker is exposed. The adsorbent materials in a multiplex adsorbent are referred to as "adsorbent species." For example, an addressable location on a probe substrate can comprise a multiplex adsorbent characterized by many different adsorbent species (*e.g*., anion exchange materials, metal chelators, or antibodies), having different binding characteristics. Substrate material itself can also contribute to adsorbing a marker and may be considered part of an "adsorbent."

"Adsorb" refers to the detectable binding between an absorbent and a marker either before or after washing with an eluant (selectivity threshold modifier) or a washing solution.

"Eluant" or "washing solution" refers to an agent that can be used to mediate adsorption of a marker to an adsorbent. Eluants and washing solutions also are referred to as "selectivity threshold modifiers." Eluants and washing solutions can be used to wash and remove unbound materials from the probe substrate surface.

"Resolve," "resolution," or "resolution of marker" refers to the detection of at least one marker in a sample. Resolution includes the detection of a plurality of markers in a sample by separation and subsequent differential detection. Resolution does not require the complete separation of a marker from all other markers in a mixture. Rather, any separation that allows the distinction between at least two markers suffices.

"Gas phase ion spectrometer" refers to an apparatus that measures a parameter which can be translated into mass-to-charge ratios of ions formed when a sample is ionized into the gas phase. Generally ions of interest bear a single charge, and mass-to-charge ratios are often simply referred to as mass.

"Mass spectrometer" refers to a gas phase ion spectrometer that includes an inlet system, an ionization source, an ion optic assembly, a mass analyzer, and a detector.

"Laser desorption mass spectrometer" refers to a mass spectrometer which uses laser as an ionization source to desorb a marker.

"Detect" refers to identifying the presence, absence or amount of the object to be detected.

"Retention" refers to an adsorption of a marker or by an adsorbent after washing with an eluant or a washing solution.

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, *e.g*., by the addition of carbohydrate residues to form glycoproteins. The terms "polypeptide," "peptide" and "protein" include glycoproteins, as well as non-glycoproteins.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g*., hydroxyproline, carboxyglutamate, and O-phosphoserine. Amino acid analogs refers to compounds that have the same basic chemical structure as a naturally occurring amino acid, *i.e.,* an carbon that is bound to a hydrogen, a carboxyl group, an amino group, and an R group, *e.g.*, homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission.

"Detectable moiety" or a "label" refers to a composition detectable by spectroscopic, photochemical, biochemical, immunochemical, or chemical means. For example, useful labels include ³²P, ³⁵S, fluorescent dyes, electron-dense reagents, enzymes (*e.g.*, as commonly used in an ELISA), biotin-streptavadin, dioxigenin, haptens and proteins for which antisera or monoclonal antibodies are available, or nucleic acid molecules with a sequence complementary to a target. The detectable moiety often generates a measurable signal, such as a radioactive, chromogenic, or fluorescent signal, that can be used to quantitate the amount of bound detectable moiety in a sample. The detectable moiety can be incorporated in or attached to a primer or probe either covalently, or through ionic, van der Waals or hydrogen bonds, *e.g.*, incorporation of radioactive nucleotides, or biotinylated nucleotides that are recognized by streptavadin. The detectable moiety may be directly or indirectly detectable. Indirect detection can involve the binding of a second directly or indirectly detectable moiety to the detectable moiety. For example, the detectable moiety can be the ligand of a binding partner, such as biotin, which is a binding partner for streptavadin, or a nucleotide sequence, which is the binding partner for a complementary sequence, to which it can specifically hybridize. The binding partner may itself be directly detectable, for example, an antibody may be itself labeled with a fluorescent molecule. The binding partner also may be indirectly detectable, for example, a nucleic acid having a complementary nucleotide sequence can be a part of a branched DNA molecule that is in turn detectable through hybridization with other labeled nucleic acid molecules. (*See, e.g.,* P. D. Fahrlander and A. Klausner, *Bio*/*Technology* 6:1165 (1988)). Quantitation of the signal is achieved by, *e.g.,* scintillation counting, densitometry, or flow cytometry.

"Antibody" refers to a polypeptide ligand substantially encoded by an immunoglobulin gene or immunoglobulin genes, or fragments thereof, which specifically binds and recognizes an epitope (*e.g.*, an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, *e.g.*, as intact immunoglobulins or as a number of well characterized fragments produced by digestion with various peptidases. This includes, *e.g.,* Fab' and F(ab)'₂ fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH₁, CH₂ and CH₃, but does not include the heavy chain variable region.

"Immunoassay" is an assay that uses an antibody to specifically bind an antigen. The immunoassay is characterized by the use of specific binding properties of a particular antibody to isolate, target, and/or quantify the antigen.

The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions may require an antibody that is selected for its specificity for a particular protein. For example, polyclonal antibodies raised to seminal basic protein from specific species such as rat, mouse, or human can be selected to obtain only those polyclonal antibodies that are specifically immunoreactive with seminal basic protein and not with other proteins, except for polymorphic variants and alleles of seminal basic protein. This selection may be achieved by subtracting out antibodies that cross-react with seminal basic protein molecules from other species. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, *Antibodies, A Laboratory Manual* (1988), for a description of immunoassay formats and conditions that can be used to determine specific immunoreactivity). Typically a specific or selective reaction will be at least twice background signal or noise and more typically more than 10 to 100 times background.

### DETAILED DESCRIPTION OF THE INVENTION

The present invention is based upon the discovery of markers that are differentially present in the samples of prostate cancer patients and subjects who have benign prostate hyperplasia, and the application of this discovery in the methods and kits for aiding a prostate cancer diagnosis. By monitoring the amount of one or more markers in a sample taken from a subject, the methods of the invention can determine the subject's pathological status. For example, one or more of these markers can be monitored to determine whether a subject, who is diagnosed with a high blood serum level of PSA, has prostate cancer or BPH. The methods and kits of the invention can also be used in addition to conventional prostate cancer testing methods to confirm the presence or absence of prostate cancer. The methods of the invention can be performed in a short amount of time using minute quantities of easily obtained biological samples such as blood, serum, urine, semen, seminal fluid, seminal plasma, or tissue extracts.

The markers of the present invention may have any suitable characteristics but have an apparent molecular weight of less than 10,000 Da. In an example, the markers are present at an elevated level in samples of prostate cancer patients compared to samples of BPH patients. These include, but are not limited to, markers having an apparent molecular weight of about 2776 Da, 4423 Da, 4480 Da, 5753 Da, 6098 Da, 6270 Da, 6998 Da, 7843 Da, 8030 Da, 8240 Da, or 8714 Da. In yet another example, the markers are present at an elevated level in samples of BPH patients compared to samples of prostate cancer patients. These include, but are not limited to, markers having an apparent molecular weight of about 2276 Da, 2530 Da, 2095 Da, 3030 Da, 3038 Da, 3224 Da, 3600 Da, 3835 Da, 3915 Da, 3933 Da, and 4175 Da. These markers may be found in a number of biological samples, and markers found in seminal plasma are preferably monitored in the methods of the invention.

Each of the markers can have particular binding characteristics which allow these markers to be enriched and measured in a sample taken from a subject under selectivity conditions that favor binding of these markers. For example, markers having an apparent molecular weight of about 2776 Da, 4423 Da, 4480 Da, 5753 Da, 6098 Da, 6270 Da, 6998 Da, 8030 Da, and 8714 Da bind to adsorbents comprising metal ions (*e.g*., nickel), indicating that these markers can have amino acids residues, such as histidine, capable of binding to metal ions. Similarly, markers having an apparent molecular weight of about 2276 Da, 2905 Da, 3038 Da, 3600 Da, 3835 Da, 3933 Da, and 4175 Da can bind to the same type of adsorbents.

Markers having an apparent molecular weight of about 2776 Da, 5753 Da, 6098 Da, 6270 Da, 6998 Da, 7843 Da, 8030 Da, and 8240 Da bind to adsorbents comprising a hydrophobic group (*e.g.,* an aliphatic C₁₆ hydrocarbon group), indicating that these markers can have amino acid residues comprising hydrophobic moieties. Similarly, markers having an apparent molecular weight of about 2276 Da, 2530 Da, 2905 Da, 3030 Da, 3224 Da, 3600 Da, and 3915 Da can bind to the same type of adsorbents.

A marker having a molecular weight of 5753 Da can further bind to adsorbents comprising a hydrophobic group and an anionic group (*e.g.*, polystyrene latex beads functionalized with a sulfonate group), indicating that this marker has basic amino acid residues and hydrophobic moieties. Among these markers, the marker having an apparent molecular weight of about 5753 Da is preferably monitored with the methods of the invention, because it can be enriched almost exclusively under certain selectivity conditions and can be detected in high quantities in samples taken from prostate cancer patients but not in samples from other subjects (*e.g.,* who have benign prostate hyperplasia).

The marker having a molecular weight of about 5753 Da is identified as seminal basic protein which is a peptide fragment generated by PSA-mediated proteolysis of semenogelin I protein (amino acid position 85-136 of semenogelin I; *see,* SEQ ID NO:1 shown in Figure 1). The seminal basic protein has a C-terminal tyrosine, and a peptide fragment of semenogelin I immediately before the seminal basic protein *(i.e.,* a fragment on the N-terminal side of seminal basic protein) has a C-terminal sequence of leucine-leucine. Both of these amino acids are known to be specific hydrolytic sites of PSA, indicating that seminal basic protein is a cleaved product generated by serine protease enzyme activity (*e.g.,* chymotrypsin-like activity) of PSA.

Seminal basic protein is abundant in the seminal plasma taken from prostate cancer patients. However, its presence is almost negligible in the seminal plasma taken from BPH patients. This indicates that the PSA in the seminal plasma of a prostate cancer patient is much more active than that in a patient with BPH, since the amount of seminal basic protein present in a sample can reflect the proteolytic activity of PSA. This conclusion is consistent with a previous finding that most PSA in BPH nodules is likely to have multiple internal cleavages resulting in much lower specific serine protease activity. *See,* Chen *et al., J. Urol.* 167: 2166-2170 (1997).

Thus, in some embodiments, cleaved products generated by PSA-mediated proteolysis in a sample from a subject can be monitored as an aid to diagnose prostate cancer, *e.g*., by measuring the amount of seminal basic protein. Monitoring the biological activity of PSA in a sample instead of measuring the amount of PSA in a sample has several advantages. First, a detected signal is amplified when the activity of PSA is measured. For example, depending on the turnover rate of the enzyme, one PSA molecule can produce many detectable cleaved products if the protein substrates (*e.g.,* semenogelin I or semenogelin II) are present in sufficient quantities. Therefore, if there is a difference in the PSA activity in the two samples, this difference can be measured using the amount of cleaved products. The amplification effect can be useful when, for example, the difference in the amount of PSA between samples is very small and is difficult to determine using conventional methods (*e.g.,* at the early stage of prostate cancer). Second, the PSA in BPH patients has much less serine protease activity than PSA in prostate cancer patients. The decreased serine protease activity can result in lower amounts of markers that are cleaved products of PSA (*e.g*., seminal basic protein). Therefore, detectable cleaved products in prostate cancer patients are likely to be greater than in BPH patents. By monitoring the markers that are cleaved products generated by PSA-mediated proteolysis, rather than monitoring the amount of PSA, the methods and kits of the invention can provide a more sensitive way to determine whether a patient has BPH or prostate cancer.

### I. METHODS FOR DETECTING MARKERS USING GAS PHASE ION SPECTROMETRY

The invention as defined herein involves comparing a plurality of markers which are differentially present in samples of a prostate cancer patient and a person who is a BPH patient. Any combination of markers described are within the scope of this aspect of this invention and can be detected. The methods for detecting these markers have many applications. According to the invention a combination of markers can be measured to differentiate between prostate cancer and BPH, and thus are useful as an aid in the diagnosis of prostate cancer in a patient. Also disclosed herein are methods for detecting these markers that can be applied to *in vitro* prostate cancer cells or *in vivo* animal models for prostate cancer to assay for and identify compounds that modulate expression of these markers.

### A. Gas Phase Ion Spectrometry Detection.

The method of the invention uses mass spectrometry, a type of gas phase low spectrometry. The following disclosure discusses gas phase ion spectrometry, but the skilled person would understand that this is applicable as mass spectrometry as referred to in the claims. A gas phase ion spectrometer can be used. This method comprises: (a) contacting a sample with a substrate comprising an adsorbent thereon under conditions to allow binding between a marker and the adsorbent; and (b) detecting the marker bound to the adsorbent by gas phase ion spectrometry.

The detection of these markers can be enhanced using certain selectivity conditions (*e.g.,* types of adsorbents used or washing solutions). In a preferred embodiment, the same or substantially the same selectivity conditions that were used to discover the markers can be used in the methods for detecting a marker in a sample. For example, a substrate comprising an adsorbent having a hydrophobic group and an anionic group (*e.g.,* polystyrene latex beads functionalized with a sulfonate group) can be used. In another example, a substrate comprising an adsorbent having a hydrophobic group (*e.g.*, an aliphatic C₁₆ hydrocarbon group) can be used. In yet another example, a substrate comprising an adsorbent having a metal ion bound to a metal chelating group (*e.g.,* nickel metal ions chelated by nitrilotriacetic acid groups) as adsorbents can be used. In some embodiments, an adsorbent can be antibodies that specifically bind to the markers (*e.g.,* seminal basic protein). Preferably, a sample is seminal plasma taken from a subject.

In one embodiment, a substrate comprising an adsorbent can be in the form of a probe, which is removably insertable into a gas phase ion spectrometer. For example, a substrate can be in the form of a strip with adsorbents on its surface. In another embodiment, a substrate comprising an adsorbent can be positioned onto another substrate to form a probe, which is removably insertable into a gas phase ion spectrometer. For example, a substrate comprising an adsorbent can be a solid phase, such as a polymeric or glass bead with a functional group for binding a marker, which can be subsequently positioned on a second substrate to form a probe. For example, the second substrate can be in the form of a strip, or a plate having a series of wells at a predetermined addressable locations. One advantage of this embodiment is that the marker can be adsorbed to the first substrate in one physical context, and transferred to the second substrate, which can then be submitted for analysis by gas phase ion spectrometry. The probe can be in any shape as long as it is removably insertable into a gas phase ion spectrometer.

The probe can also be adapted for use with inlet systems and detectors of a gas phase ion spectrometer. For example, the probe can be adapted for mounting in a horizontally and/or vertically translatable carriage that horizontally and/or vertically moves the probe to a successive position without requiring repositioning of the probe by hand.

The probe substrate is preferably made of a material that is capable of supporting adsorbents. For example, the probe substrate material can include, but is not limited to, insulating materials (*e.g.*, glass, ceramic), semi-insulating materials (*e.g.*, silicon wafers), or electrically conducting materials (*e.g.*, metals, such as nickel, brass, steel, aluminum, gold, or electrically conductive polymers), organic polymers, biopolymers, or any combinations thereof.

The probe substrate surface can be conditioned to bind markers. For example, in one embodiment, the surface of the probe substrate can be conditioned (*e.g*., chemically or mechanically such as roughening) to place adsorbents on the surface. The adsorbent comprises functional groups for binding with a marker. In some embodiments, the substrate material itself can also contribute to adsorbent properties and may be considered part of an "adsorbent."

Any number of different adsorbents can be used as long as they have binding characteristics suitable for binding the markers of the present invention. The adsorbents can comprise a hydrophobic group, a hydrophilic group, a cationic group, an anionic group, a metal ion chelating group, or antibodies which specifically bind to antigens, or a combination thereof (sometimes referred to as "a mixed mode" adsorbent). Exemplary adsorbents comprising a hydrophobic group include matrices having aliphatic hydrocarbons, *e.g.,* C₁-C₁₈ aliphatic hydrocarbons and matrices having aromatic hydrocarbon functional group such as phenyl groups. Exemplary adsorbents comprising a hydrophilic group include silicon oxide (*i.e.*, glass), or hydrophilic polymers such as polyethylene glycol, dextran, agarose, or cellulose. Exemplary adsorbents comprising a cationic group include matrices of secondary, tertiary or quaternary amines. Exemplary adsorbents comprising an anionic group include matrices of sulfate anions (SO₃⁻) and matrices of carboxylate anions (*i.e.,* COO⁻) or phosphate anions (OPO₃⁻). Exemplary adsorbents comprising metal chelating groups include organic molecules that have one or more electron donor groups which form coordinate covalent bonds with metal ions, such as copper, nickel, cobalt, zinc, iron, and other metal ions such as aluminum and calcium. Exemplary adsorbents comprising an antibody include antibodies that are specific for any one of the markers provided herein. In preferred embodiments, adsorbents are substantially similar to or the same as the adsorbents which were used to enrich and identify the markers.

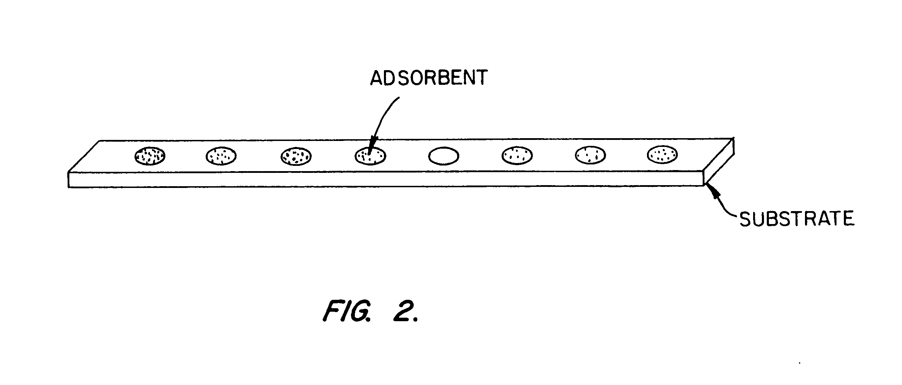

Adsorbents can be placed on the probe substrate in continuous or discontinuous patterns. If continuous, one or more adsorbents can be placed on the substrate surface. If multiple types of adsorbents are used, the substrate surface can be coated such that one or more binding characteristics vary in one or two-dimensional gradient. If discontinuous, plural adsorbents can be placed in predetermined addressable locations on the substrate surface. The addressable locations can be arranged in any pattern, but are preferably in regular pattern, such as lines, orthogonal arrays, or regular curves (*e.g*., circles). Each addressable location may comprise the same or different adsorbent. In Figure 2, a probe comprising discontinuous spots of adsorbents is shown.

The probes can be produced using any suitable methods depending on the selection of substrate materials and/or adsorbents. For example, the surface of a metal substrate can be coated with a material that allows derivitization of the metal surface. More specifically, a metal surface can be coated with silicon oxide, titanium oxide or gold. Then surface can be derivatized with a bifunctional linker, one end of which can covalently bind with a functional group on the surface and the other end of which can be further derivatized with groups that function as an adsorbent. In another example, a porous silicon surface generated from crystalline silicon can be chemically modified to include adsorbents for binding markers. In yet another example, adsorbents with a hydrogel backbone can be formed directly on the substrate surface by *in situ* polymerizing a monomer solution which comprises, *e.g*., substituted acrylamide monomers, substituted acrylate monomers, or derivatives thereof comprising a functional group of choice as an adsorbent.

Probes suitable for use in the invention are also described in, *e.g.,* WO98/59361, U.S.S.N. 60/131,652, filed April 29, 1999, and Wei *et al., Nature* 399:243-246(1999).

The probe substrate comprising an adsorbent contacts a sample. The sample is preferably a biological fluid sample. Examples of biological fluid samples include blood, serum, urine, semen, seminal fluid, seminal plasma or tissue extracts. In a preferred embodiment, the biological fluid comprises seminal plasma.

The sample can be solubilized in or admixed with an eluant. The probe substrate comprising an adsorbent then contacts the solution using any techniques including bathing, soaking, dipping, spraying, washing over, or pipetting, *etc.* Generally, a volume of sample containing from a few atomoles to 100 picomoles of marker in about 1 µl to 500 µl is sufficient for binding to the adsorbent.

The sample can contact the probe substrate comprising an adsorbent for a period of time sufficient to allow the marker to bind to the adsorbent. Typically, the sample and the substrate comprising the adsorbent are contacted for a period of between about 30 seconds and about 12 hours, and preferably, between about 30 seconds and about 15 minutes.

The temperature at which the sample contacts the probe substrate comprising an adsorbent can be a function of the particular sample and the selected probe. Typically, the sample is contacted to the probe substrate under ambient temperature and pressure conditions. For some samples, however, modified temperature (typically 4°C through 37°C), and pressure conditions can be desirable, which conditions are determinable by those skilled in the art.

After the probe substrate comprising an adsorbent contacts the sample or sample solution, it is preferred that unbound materials on the probe substrate surface are washed out so that only the bound materials remain on the substrate surface. Washing a probe substrate surface can be accomplished by, *e.g.*, bathing, soaking, dipping, rinsing, spraying, or washing the substrate surface with an eluant or a washing solution. A microfluidics process is preferably used when a washing solution such as an eluant is introduced to small spots of adsorbents on the probe. Typically, the washing solution can be at a temperature of between 0°C and 100°C, preferably between 4°C and 37°C.

Any suitable washing solutions or eluants can be used to wash the probe substrate surface. For example, organic solutions or aqueous solutions can be used. Preferably, an aqueous solution is used. Exemplary aqueous solutions include a HEPES buffer, a Tris buffer, a phosphate buffered saline, *etc.* The selection of a particular washing solution or an eluant is dependent on other experimental conditions (*e.g*., types of adsorbents used or markers to be detected), and can be determined by those of skill in the art. For example, if a probe comprising a hydrophobic group and a sulfonate group as adsorbents (*e.g*., SCX1 ProteinChip^{®} array) is used, then an aqueous solution, such as a HEPES buffer, may be preferred. In another example, if a probe comprising a metal binding group as an adsorbent (*e.g.,* Ni(II) ProteinChip^{®} array) is used, then an aqueous solution, such as a phosphate buffered saline, may be preferred. In yet another example, if a probe comprising a hydrophobic group (*e.g.,* H4 ProteinChip^{®}) is used, then water may be preferred as a washing solution.

Optionally, an energy absorbing molecule (*e.g.,* in solution) can be applied to markers or other substances bound on the probe substrate surface. Spraying, pipetting, or dipping can be used. This can be done after unbound materials are washed off of the probe substrate surface. An energy absorbing molecule refers to a molecule that absorbs energy from an energy source in a gas phase ion spectrometer, thereby assisting desorption of markers or other substances from a probe surface. Exemplary energy absorbing molecules include cinnamic acid derivatives, sinapinic acid and dihydroxybenzoic acid.

After the marker is bound to the probe, it is detected using gas phase ion spectrometry. Markers or other substances bound to the adsorbents on the probes can be analyzed using a gas phase ion spectrometer. This includes, *e.g.*, mass spectrometers, ion mobility spectrometers, or total ion current measuring devices. The quantity and characteristics of the marker can be determined using gas phase ion spectrometry. Other substances in addition to the marker of interest can also be detected by gas phase ion spectrometry.

According to the invention, a mass spectrometer is used. In a typical mass spectrometer, a probe with a marker is introduced into an inlet system of the mass spectrometer. The marker is then ionized by an ionization source such as a laser, fast atom bombardment, or plasma. The generated ions are collected by an ion optic assembly, and then a mass analyzer disperses and analyzes the passing ions. The ions exiting the mass analyzer are detected by a detector. The detector then translates information of the detected ions into mass-to-charge ratios. Detection of the presence of a marker or other substances will typically involve detection of signal intensity. This, in turn, can reflect the quantity and character of a marker bound to the probe.

In a preferred embodiment, a laser desorption time-of-flight mass spectrometer is used with the probe of the present invention. In laser desorption mass spectrometry, a probe with a bound marker is introduced into an inlet system. The marker is desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ionization and impact can be used to identify the presence or absence of molecules of specific mass. As any person skilled in the art understands, any of these components of the laser desorption time-of-flight mass spectrometer can be combined with other components described herein in the assembly of mass spectrometer that employs various means of desorption, acceleration, detection, measurement of time, *etc.*

In another embodiment, an ion mobility spectrometer can be used to detect and characterize a marker. The principle of ion mobility spectrometry is based on different mobility of ions. Specifically, ions of a sample produced by ionization move at different rates, due to their difference in, *e.g*., mass, charge, or shape, through a tube under the influence of an electric field. The ions (typically in the form of a current) are registered at the detector which can then be used to identify a marker or other substances in the sample. One advantage of ion mobility spectrometry is that it can operate at atmospheric pressure.

In yet another embodiment, a total ion current measuring device can be used to detect and characterize markers. This device can be used when the probe has a surface chemistry that allows only a single type of marker to be bound. When a single type of marker is bound on the probe, the total current generated from the ionized marker reflects the nature of the marker. The total ion current produced by the marker can then be compared to stored total ion current of known compounds. Characteristics of the marker can then be determined.

Data generated by desorption and detection of markers can be analyzed with the use of a programmable digital computer. The computer program generally contains a readable medium that stores codes. Certain code can be devoted to memory that includes the location of each feature on a probe, the identity of the adsorbent at that feature and the elution conditions used to wash the adsorbent. Using this information, the program can then identify the set of features on the probe defining certain selectivity characteristics (*e.g.,* types of adsorbent and eluants used). The computer also contains code that receives as input, data on the strength of the signal at various molecular masses received from a particular addressable location on the probe. This data can indicate the number of markers detected, optionally including the strength of the signal and the determined molecular mass for each marker detected.

Data analysis can include the steps of determining signal strength (*e.g.,* height of peaks) of a marker detected and removing "outerliers" (data deviating from a predetermined statistical distribution). For example, the observed peaks can be normalized, a process whereby the height of each peak relative to some reference is calculated. For example, a reference can be background noise generated by instrument and chemicals (*e.g.,* energy absorbing molecule) which is set as zero in the scale. Then the signal strength detected for each marker or other substances can be displayed in the form of relative intensities in the scale desired (*e.g*., 100). Alternatively, a standard may be admitted with the sample so that a peak from the standard can be used as a reference to calculate relative intensities of the signals observed for each marker or other markers detected.

The computer can transform the resulting data into various formats for displaying: In one format, referred to as "spectrum view or retentate map," a standard spectral view can be displayed, wherein the view depicts the quantity of marker reaching the detector at each particular molecular weight. In another format, referred to as "peak map," only the peak height and mass information are retained from the spectrum view, yielding a cleaner image and enabling markers with nearly identical molecular weights to be more easily seen. In yet another format, referred to as "gel view," each mass from the peak view can be converted into a grayscale image based on the height of each peak, resulting in an appearance similar to bands on electrophoretic gels. In yet another format, referred to as "3-D overlays," several spectra can be overlayed to study subtle changes in relative peak heights. In yet another format, referred to as "difference map view," two or more spectra can be compared, conveniently highlighting unique markers and markers which are up- or down-regulated between samples. Marker profiles (spectra) from any two samples may be compared visually.

Using any of the above display formats, it can be readily determined from the signal display whether a marker having a particular molecular weight (*e.g.,* about 2776 Da, 4423 Da, 4480 Da, 5753 Da, 6098 Da, 6270 Da, 6998 Da, 7843 Da, 8030 Da, 8240 Da, or 8714 Da) is detected from a sample. Moreover, from the strength of signals, the amount of markers bound on the probe surface can be determined.

### B. Immunoassay Detection

Also disclosed herein is a detection method, wherein an immunoassay can be used to qualitatively or quantitatively detect and analyze markers in a sample. This method comprises: (a) providing an antibody that specifically binds to a marker, wherein the marker is a polypeptide which is differentially present in samples of a prostate cancer patient and a subject who does not have prostate cancer (*e.g.*, BPH patients); (b) contacting a sample with the antibody; and (c) detecting the presence of a complex of the antibody bound to the marker in the sample.

To prepare an antibody that specifically binds to a marker, purified markers or their nucleic acid sequences can be used. For the marker having a molecular weight of about 5753 Da, a seminal basic protein, nucleic acid and amino acid sequences are available. *See, e.g.,* Figure 1 and Lilja and Jeppsson, *Febs. Lett.* 182:181-184 (1985). Nucleic acid and amino acid sequences for other markers can be obtained by further characterization of these markers. For example, each marker can be peptide mapped with a number of enzymes (*e.g*., trypsin, V8 protease, *etc.*). The molecular weights of digestion fragments from each marker can be used to search the databases, such as SwissProt database, for sequences that will match the molecular weights of digestion fragments generated by various enzymes. Using this method, the nucleic acid and amino acid sequences of other markers can be identified if these markers are known proteins in the databases.

Alternatively, the proteins can be sequenced using protein ladder sequencing. Protein ladders can be generated by, for example, fragmenting the molecules and subjecting fragments to enzymatic digestion or other methods that sequentially remove a single amino acid from the end of the fragment. Methods of preparing protein ladders are described, for example, in International Publication WO 93/24834 (Chait *et al.*) and United States Patent 5,792,664 (Chait *et al.*). The ladder is then analyzed by mass spectrometry. The difference in the masses of the ladder fragments identify the amino acid removed from the end of the molecule.

If the markers are not known proteins in the databases, nucleic acid and amino acid sequences can be determined with knowledge of even a portion of the amino acid sequence of the marker. For example, degenerate probes can be made based on the N-terminal amino acid sequence of the marker. These probes can then be used to screen a genomic or cDNA library created from a sample from which a marker was initially detected. The positive clones can be identified, amplified, and their recombinant DNA sequences can be subcloned using techniques which are well known. *See, e.g., Current Protocols for Molecular Biology* (Ausbel *et al.,* Green Publishing Assoc. and Wiley-Interscience 1989) *and Molecular Cloning: A Laboratory Manual,* 2nd Ed. (Sambrook *et al.,* Cold Spring Harbor Laboratory, NY 1989).

Using the purified markers or their nucleic acid sequences, antibodies that specifically bind to a marker can be prepared using any suitable methods known in the art. *See, e.g.,* Coligan, *Current Protocols in Immunology* (1991); Harlow & Lane, *Antibodies: A Laboratory Manual* (1988); Goding, *Monoclonal Antibodies: Principles and Practice* (2d ed. 1986); and Kohler & Milstein, *Nature* 256:495-497 (1975). Such techniques include, but are not limited to, antibody preparation by selection of antibodies from libraries of recombinant antibodies in phage or similar vectors, as well as preparation of polyclonal and monoclonal antibodies by immunizing rabbits or mice (*see, e.g.,* Huse *et al., Science* 246:1275-1281 (1989); Ward *et al., Nature* 341:544-546 (1989)).

After the antibody is provided, a marker can be detected and/or quantified using any of a number of well recognized immunological binding assays *(see, e.g.,* U.S. Patents 4,366,241; 4,376,110; 4,517,288; and 4,837,168). Useful assays include, for example, an enzyme immune assay (EIA) such as enzyme-linked immunosorbent assay (ELISA), a radioimmune assay (RIA), a Western blot assay, or a slot blot assay. For a review of the general immunoassays, *see also, Methods in Cell Biology: Antibodies in Cell Biology,* volume 37 (Asai, ed. 1993); *Basic and Clinical Immunology* (Stites & Terr, eds., 7th ed. 1991); Harlow & Lane, *Antibodies, A Laboratory Manual* (1988).

Generally, a sample obtained from a subject can be contacted with the antibody that specifically binds the marker. Optionally, the antibody can be fixed to a solid support to facilitate washing and subsequent isolation of the complex, prior to contacting the antibody with a sample. Examples of solid supports include glass or plastic in the form of, *e.g*., a microtiter plate, a stick, a bead, or a microbead. Antibodies can also be attached to a probe substrate or ProteinChip^{®} array described above. The sample is preferably a biological fluid sample taken from a subject. Examples of biological fluid samples include blood, serum, urine, semen, seminal fluid, seminal plasma, or tissue extracts. In a preferred embodiment, the biological fluid comprises seminal plasma. The sample can be diluted with a suitable eluant before contacting the sample to the antibody.

After incubating the sample with antibodies, the mixture is washed and the antibody-marker complex formed can be detected. This can be accomplished by incubating the washed mixture with a detection reagent. This detection reagent may be, *e.g*., a second antibody which is labeled with a detectable label. Exemplary detectable labels include magnetic beads (*e.g.,* DYNABEADS™), fluorescent dyes, radiolabels, enzymes (*e.g*., horse radish peroxide, alkaline phosphatase and others commonly used in an ELISA), and colorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10°C to 40°C.

Immunoassays can be used to determine the presence or absence of a marker in a sample as well as the quantity of a marker in a sample. First, a test amount of a marker in a sample can be detected using the immunoassay methods described above. If a marker is present in the sample, it will form an antibody-marker complex with an antibody that specifically binds the marker under suitable incubation conditions described above. The amount of an antibody-marker complex can be determined by comparing to a standard. A standard can be, *e.g*., a known compound or another protein known to be present in a sample. As noted above, the test amount of marker need not be measured in absolute units, as long as the unit of measurement can be compared to a control.

The methods for detecting these markers in a sample have many applications. For example, one or more markers can be measured to aid prostate cancer diagnosis or prognosis. In another example, the methods for detection of the markers can be used to monitor responses in a subject to cancer treatment. In another example, the methods for detecting markers can be used to assay for and to identify compounds that modulate expression of these markers *in vivo* or *in vitro.*

### II. METHODS FOR DIAGNOSING PROSTATE CANCER USING TEST AMOUNT OF MARKERS

The invention as defined herein provides methods for aiding a prostate cancer diagnosis using a plurality of markers which are differentially present in samples of a prostate cancer patient and a subject who is a BPH patient. Any combination of markers described above can be used for aiding prostate cancer diagnosis. Compared to the current prostate cancer tests available, the present methods provide a quick and simple way to differentiate if a subject has prostate cancer or BPH, and thus aiding a prostate cancer diagnosis. The methods comprise: (a) determining a test amount of a marker in a sample from the subject; and (b) determining whether the test amount is a diagnostic amount consistent with a diagnosis of prostate cancer.

In step (a), a test amount of a marker in a sample from a subject is determined. Any suitable samples can be obtained from a subject. Preferably, a sample is a biological fluid sample taken from a subject being tested. Examples of biological fluid samples include blood, serum, urine, semen, seminal fluid, seminal plasma or tissue extracts. In a preferred embodiment, the biological fluid comprises seminal plasma, because seminal plasma is a bodily fluid which is physically proximate to the prostate and other tissue first affected by cancerous cells. Thus, seminal plasma will likely contain proteins that are distinct to prostate cancer or proteins that are up-regulated or down-regulated. Moreover, testing a seminal plasma sample does not require an invasive procedure, such as inserting a needle into a patient.

After a sample is obtained, any suitable method can be used to determine a test amount of the marker in a sample from a subject being tested. For example, gas phase ion spectrometry or an immunoassay can be used.

Mass spectrometry is used to determine a test amount of markers in a sample from a subject. First, one or more markers can be detected with gas phase ion spectrometry using the methods described above. After the marker is detected by a gas phase ion spectrometer, the test amount of marker can be determined. For example, a signal is displayed at the molecular weight of the marker of interest. Based on the strength or magnitude of the displayed signal, the amount of marker in a sample being tested can be determined. It is noted that the test amount of marker in a sample need not be measured in absolute units, but can be in relative units as long as it can be compared qualitatively or quantitatively to a control amount of a marker. For example, as described above, the amount of the marker detected can be displayed in terms of relative intensity based on the background noise. Preferably, the test amount and the control amount of markers are measured under the same conditions.

If desired, the absolute amount of a marker can be determined by calibration. For example, a purified marker such as seminal basic protein can be added in increasing amounts to different spots of adsorbents on the probe surface. Then peaks from each spot can be obtained and plotted in a graph against the concentration of seminal basic protein at each spot. From the peak intensity vs. concentration plot, the absolute amount of a marker in any sample being tested can be determined.

Also disclosed herein is an immunoassay that can be used to determine a test amount of a marker in a sample from a subject. First, a test amount of a marker in a sample can be detected using the immunoassay methods described above. If a marker is present in the sample, it will form an antibody-marker complex with an antibody that specifically binds the marker under suitable incubation conditions described above. The amount of an antibody-marker complex can be determined by comparing to a standard. As noted above, the test amount of marker need not be measured in absolute units, as long as the unit of measurement can be compared to a control amount.

After a test amount of marker is determined using either method, then based on the test amount, it can be determined whether a subject has prostate cancer. This determination can be made by any suitable methods. For example, the test amount can be compared to a control amount which can be a value or a range of values determined as follows.

The control amount is an amount of a markers present in comparable samples from BPH patients. The control amount is measured under the same or substantially similar experimental conditions as in measuring the test amount. For example, if a test sample is obtained from a subject's seminal plasma and a marker is detected using a particular probe, then a control amount of the markers is preferably determined from a seminal plasma sample of a BPH patient using the same probe. It is preferred that the control amount of markers is determined based upon a significant number of samples from subjects who do not have prostate cancer (BPH patients) so that it reflects variations of the marker amounts in that population. If the test amount of marker is significantly increased compared to the control amount of marker which is known to be elevated in samples of prostate cancer patients (*e.g.,* 5753 Da), then it can be a positive indication that a subject being tested has prostate cancer. For example, if the test amount is increased by 1.5 fold, preferably by 2 fold, more preferably by 5 fold, or most preferably by 10 fold compared to the control amount, then the test amount is a diagnostic amount which is consistent with a diagnosis of prostate cancer. The converse would apply for markers that are known to be elevated in the samples of BPH patients than prostate cancer patients (*e.g.,* 3600 Da).

A control amount can be an amount of a marker present in comparable samples from a prostate cancer patient. Again, it is preferred that the control amount of a marker is determined based upon a significant number of samples taken from prostate cancer patients so that it reflects variations of the marker amounts in that population. If the test amount of the marker is about the same as the control amount of the marker, then it can be a positive indication that a subject being tested has prostate cancer.

A control amount can be an amount of a marker present in comparable samples from a normal person (*i.e*., who is known to be free of prostate cancer and BPH). It is preferred that the control amount of marker is determined based upon a significant number of samples taken from normal persons so that it reflects variations of the marker amounts in that population. If the control amount of a particular marker (*e.g*., 5753 Da) is significantly lower than the amount of the same marker present in comparable samples of prostate cancer patients, then this marker can be used to diagnose prostate cancer and rule out BPH in a single test. In such a case, if the test amount of marker is significantly increased compared to the control amount of marker, then it can be a positive indication that a subject being tested has prostate cancer. For example, if the test amount is increased by 1.5 fold, preferably by 2 fold, more preferably by 5 fold, most preferably by 10 fold compared to the control amount, then the test amount is a diagnostic amount which is consistent with a diagnosis of prostate cancer. The converse would apply for markers that are known to be elevated in the samples of BPH patients than prostate cancer patients (*e.g*., 3600 Da).

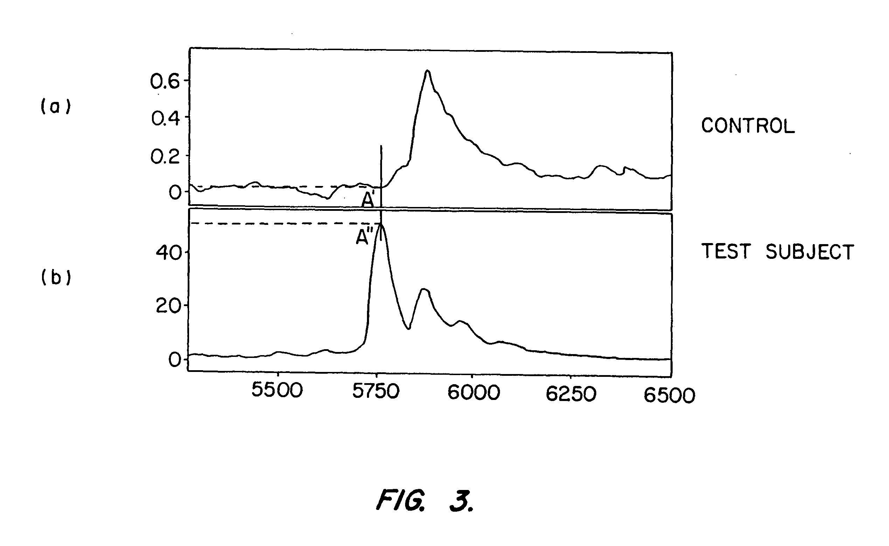

An illustration of a step for determining whether the test amount is a diagnostic amount for prostate cancer can be described with reference to Figures 3(a) and 3(b). Figure 3(a) is an exemplary mass spectra of a control representing maker amounts in BPH patients' samples measured using SCX1 ProteinChip^{®} which is capable of detecting a marker having an apparent molecular weight of about 5753 Da (seminal basic protein). As shown in Figure 3(a), peak A' has a value of less than about 0.1 at molecular weight of 5753 Da and corresponds to the amount of seminal basic protein in a patient with BPH. Figure 3(b) is a mass spectra of a test sample taken from a subject. As shown in Figure 3(b), peak A" is at the same molecular weight as peak A' in Figure 3(a) and corresponds to the test amount of seminal basic protein in the test sample. Compared to peak A' shown in Figure 3(a) which represents the control amount of the marker in BPH patients, peak A" shown in Figure 3(b) is significantly higher and has a value of about 52. Since peak A" representing the test amount of marker in the subject's sample is significantly greater than peak A' representing the control value obtained from the BPH patients' sample, it can be determined that the sample used to produce a mass spectra in Figure 3(b) was taken from a subject having prostate cancer.

Data generated by mass spectrometry can then be analyzed by a computer software. The software can comprise code that converts signal from the mass spectrometer into computer readable form. The software also can include code that applies an algorithm to the analysis of the signal to determine whether the signal represents a "peak" in the signal corresponding to a marker of this invention, or other useful markers. The software also can include code that executes an algorithm that compares signal from a test sample to a typical signal characteristic of "normal" and prostate cancer and determines the closeness of fit between the two signals. The software also can include code indicating which the test sample is closest to, thereby providing a probable diagnosis.

### III. KITS

Also described are kits for aiding a diagnosis of prostate cancer, wherein the kits can be used to detect the markers of the present invention. For example, the kits can be used to detect any one or combination of markers described above, which markers are differentially present in samples of a prostate cancer patient and a BPH patient. The described kits have many applications. For example, the kits can be used to differentiate if a subject has prostate cancer or BPH, thus aiding a prostate cancer diagnosis. In another example, the kits can be used to identify compounds that modulate expression of the markers in *in vitro* prostate cells or *in vivo* animal models for prostate cancer.

A kit may comprise: (a) a substrate comprising an adsorbent thereon, wherein the adsorbent is suitable for binding a marker, and (b) a washing solution or instructions for making a washing solution, wherein the combination of the adsorbent and the washing solution allows detection of the marker using gas phase ion spectrometry. Such kits can be prepared from the materials described above, and the previous discussion of these materials (*e.g.,* probe substrates, adsorbents, washing solutions, *etc.*) is fully applicable to this section and need not be repeated.

The kit may comprise a first substrate comprising an adsorbent thereon (*e.g.*, a particle functionalized with an adsorbent) and a second substrate onto which the first substrate can be positioned to form a probe which is removably insertable into a gas phase ion spectrometer. In other embodiments, the kit may comprise a single substrate which is in the form of a removably insertable probe with adsorbents on the substrate.

Optionally, the kit can further comprise instructions for suitable operational parameters in the form of a label or a separate insert. For example, the kit may have standard instructions informing a consumer how to wash the probe after a sample of seminal plasma is contacted on the probe.

A kit may comprise (a) an antibody that specifically binds to a marker; and (b) a detection reagent. Such kits can be prepared from the materials described above, and the previous discussion regarding the materials (*e.g.,* antibodies, detection reagents, immobilized supports, *etc*.) is fully applicable to this section and need not be repeated.

The kit may optionally further comprise a standard or control information so that the test sample can be compared with the control information standard to determine if the test amount of a marker detected in a sample is a diagnostic amount consistent with a diagnosis of prostate cancer.

### EXAMPLES

The following examples are offered by way of illustration, not by way of limitation.

### A. Identification of Markers Using Ni(II) Protein Chip^{®} Array

Ni(II) ProteinChip^{®} Array was fabricated as follows. The surface of a metal substrate was conditioned by etching via laser (*e.g.,* Quantred Company, Galaxy model, ND-YAG Laser, using emission line of 1.064 nm, power of 30-35 watts with a laser spot size of 0.005 inches, the laser source to surface distance of 12-14 inches; with a rate of scan of about 25 per mm per second). Then the etched surface of the metal substrate was coated with a glass-coating. 5-Methacylamido-2-(N,N-biscarboxymethaylamino)pentanoic acid (7.5 wt%), acryloytri(hydroxymethyl)methylamine (7.5 wt%) and N,N'-methylenebisacrylamide (0.4 wt%) were photo-polymerized using (-)-riboflavin (0.02 wt%) as a photo-initiator. The monomer solution was deposited onto a rough etched, glass coated substrate (0.4 µL, twice) and was irradiated for 5 minutes with a near UV exposure system (Hg short arc lamp, 20 mW/cm² at 365 nm). The surface was washed with a solution of sodium chloride (1 M), and then the surface was washed twice with deionized water. The surface was treated with a solution of NiSO₄ (50 mM, 10 mL/spot) and mixed on a high frequency mixer for 10 minutes. After removing the NiSO4 solution, the treatment process was repeated. Finally, the surface was washed with a stream of deionized water (15 sec/chip), thereby providing the Ni(II) ProteinChip^{®} array.

Seminal plasma samples were obtained from a patient with prostate cancer and a patient with BPH. From each sample, 2 µl seminal plasma is added to 3 µl of 9.5 M urea, 2% CHAPS, 100 mM TrisHCl, pH 9.0. The mixture was vortexed in the cold for 5 min. This mixture was further diluted 1/10 into 50 mM HEPES, pH 7.4. The diluted sample solution was added to a spot of adsorbent on the Ni(II) ProteinChip^{®} array (Ciphergen Biosystems, Inc., Fremont, CA). Ni(II) ProteinChip^{®} array comprises, as an adsorbent, a nickel metal ion chelated by nitrolotriacetic acid groups covalently linked to the hydrogel backbone. The sample solution was incubated for 15 min with shaking in a humidity chamber. The ProteinChip^{®} array was washed with phosphate buffered saline, pH 7.2. 0.3 µl of CHCA (saturated solution in acetonitrile, trifluoroacetic acid) was added to the spot containing the sample, and the ProteinChip^{®} array was read in Protein Biology System II mass spectrometer (Ciphergen Biosystems, Inc.).

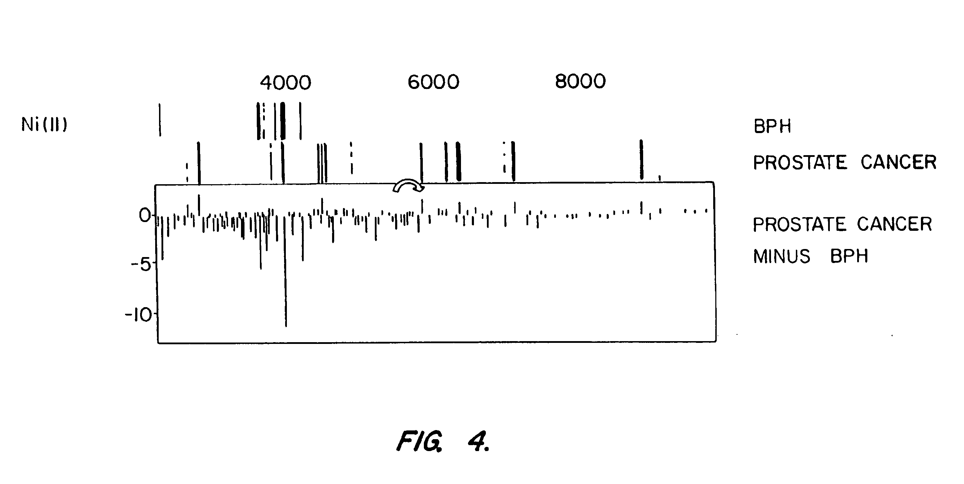

Figure 4 illustrates the results. The top panel shows the proteins from the sample of the BPH patient that are desorbed from the Ni(II) ProteinChip^{®} array in the gel view format, in which each protein detected is represented as a band that corresponds to a particular molecular weight. The middle panel shows the proteins from the sample of the prostate cancer patient that are desorbed from the Ni(II) ProteinChip^{®} array in the gel view format. The bottom panel shows a difference map between the two samples. The vertical lines above zero represent proteins that are present at an elevated level in the sample from the prostate cancer patient compared to the sample from the BPH patient. Conversely, the vertical lines below zero represent proteins that are present at an elevated level in the sample from the BPH patient compared to the sample from the prostate cancer patient. The higher the vertical line, greater the difference in the quantity of the protein found between the two samples

As shown in Figure 4, a number of proteins were found to be very abundant in the sample from the prostate cancer patient than in the sample from the BPH patient. For example, proteins of apparent molecular weight of about 2776 Da, 4423 Da, 4480 Da, 5753 Da, 6098 Da, 6270 Da, 6998 Da, 8030 Da, and 8714 Da were found to be very abundant in a sample from a prostate cancer patient than a sample from a BPH patient. Moreover, a number of proteins were found to be very abundant in the samples from the BPH patient than in the sample from the prostate cancer patient. For examples, proteins having an apparent molecular weight of about 2276 Da, 2905 Da, 3038 Da, 3600 Da, 3835 Da, 3933 Da, and 4175 Da were found to be very abundant in a sample from the BPH patient than a sample from the prostate cancer patient.

### B. Identification of Markers Using H4 ProteinChip^{®} Array

H4 ProteinChip^{®} array was fabricated from SiO₂ coated aluminum substrates. In the process, the aluminum substrates were exposed to a 2% (v/v) solution of 3-aminopropyltriethoxyxilane in ethyl alcohol for one hour. After rinsing the substrate surface with ethanol, the substrates were dried (120 °C, 20 min). The aminosilanated aluminum substrates were then reacted with a solution of poly(octadecene-alt-maleic anhydride) in ethyleneglycol-dmethylether and triethylamine for 4 hours. The activated aluminum substrates were then rinsed with ethylene glycol dimethyl ether and air dried, thereby providing a H4 ProteinChip^{®} array.

Seminal plasma samples that were prepared using substantially the same solutions and procedures described above. The diluted sample solution was added to a spot of adsorbent on H4 ProteinChip^{®} array (Ciphergen Biosystems, Inc., Fremont, CA). The H4 ProteinChip^{®} array comprises as an adsorbent an aliphatic (C₁₆) hydrocarbon hydrophobic surface. The mixture was incubated for 15 min with shaking in a humidity chamber. The sample solution was air dried on the spot. The ProteinChip^{®} array was washed with water. 0.3 µl of CHCA (saturated solution in acetonitrile, trifluoroacetic acid) was added to the spot containing the sample, and the ProteinChip^{®} array was read in Protein Biology System II™ mass spectrometer (Ciphergen Biosystems, Inc., Fremont, CA).

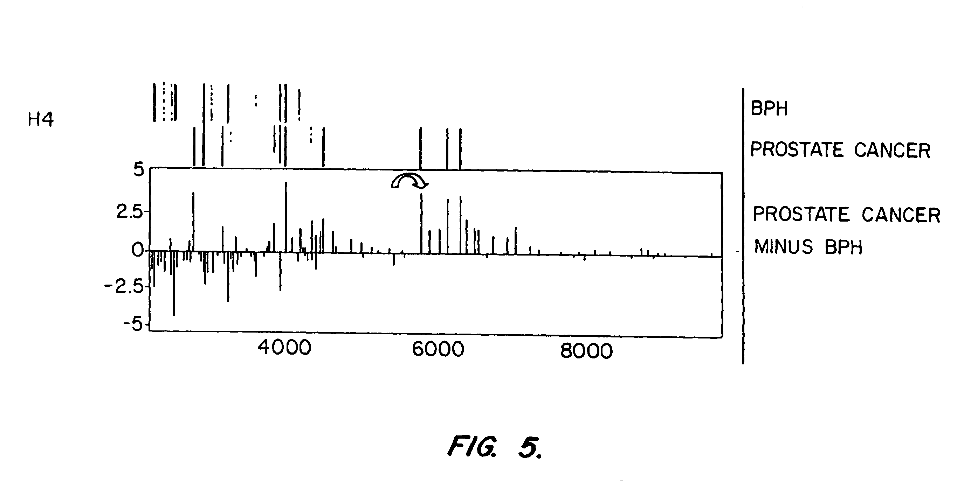

Figure 5 illustrates the results. The top panel shows the proteins from the sample of the BPH patient that were desorbed from the H4 ProteinChip^{®} array in the gel view format, in which each protein detected was represented as a band that corresponds to a particular molecular weight. The middle panel shows the proteins from the sample of the prostate cancer patient that were desorbed from the H4 ProteinChip^{®} array in the gel view format. The bottom panel shows a difference map between the two samples. The vertical lines above zero represents proteins that are present at an elevated level in the sample from the prostate cancer patient compared to the sample from the BPH patient.

As shown in Figure 5, a number of proteins were found to be very abundant in the sample from the prostate cancer patient than in the sample from the BPH patient. For example, proteins having an apparent molecular weight of about 2776 Da, 5753 Da, 6098 Da, 6270 Da, 6998 Da, 7843 Da, 8030 Da, and 8240 Da were found to be very abundant in the sample from the prostate cancer patient than the sample from the BPH patient. It is noted that proteins having an apparent molecular weight of about 2776 Da, 6098 Da, 6270 Da, 6998 Da, and 8030 Da were also bound and detected using the Ni(II) ProteinChip^{®} array.

Moreover, a number of proteins were found to be very abundant in the sample from the BPH patient than in the sample from the prostate cancer patient. For example, proteins having an apparent molecular weight of about 2276 Da, 2530 Da, 2905 Da, 3030 Da, 3224 Da, 3600 Da, and 3915 Da were bound to be very abundant in the sample from the prostate cancer patient than the sample from the BPH patient. It is noted that proteins having an apparent molecular weight of about 2276 Da, 2905 Da, and 3600 Da were also bound and detected using the Ni(II) ProteinChip^{®} array.

### C. Marker Identified Using SCXI ProteinChip^{®} Array

The SCX1 ProteinChip^{®} Array were fabricated from SiO₂ coated aluminum substrates. In the process, a suspension of sulfonated polystyrene microspheres in distilled water was deposited onto the surface of the chip (2 mL/spot, two times). After air drying (room temperature, 5 minutes), the surface is washed once with 100mM ammonium citrate (pH 4.5), once with 100mM ammonium bicarbonate (pH 8.0) and once with deinoized water. The chip was air dried.

Seminal plasma samples were obtained from a patient with prostate cancer and a patient with BPH. From each sample, 2 µl seminal plasma is added to 3 µl of 9.5 M urea, 2% CHAPS, 100 mM TrisHCl, pH 9.0. The mixture was vortexed in the cold for 5 min. This mixture was further diluted 1/10 into 50 mM HEPES, pH 7.4. 4 µl aliquot of this diluted solution was added to a spot of SCX1 ProteinChip^{®} array (Ciphergen Biosystems, Inc.), and incubated for 15 min with shaking in a humidity chamber. The sample was removed and the ProteinChip^{®} array was washed with 4 µl of 50 mM HEPES two times. 0.3 µl of CHCA (saturated solution in acetonitrile, trifluoroacetic acid) was added and the ProteinChip^{®} array was read in Protein Biology System II™ mass spectrometer (Ciphergen Biosystems, Inc.).

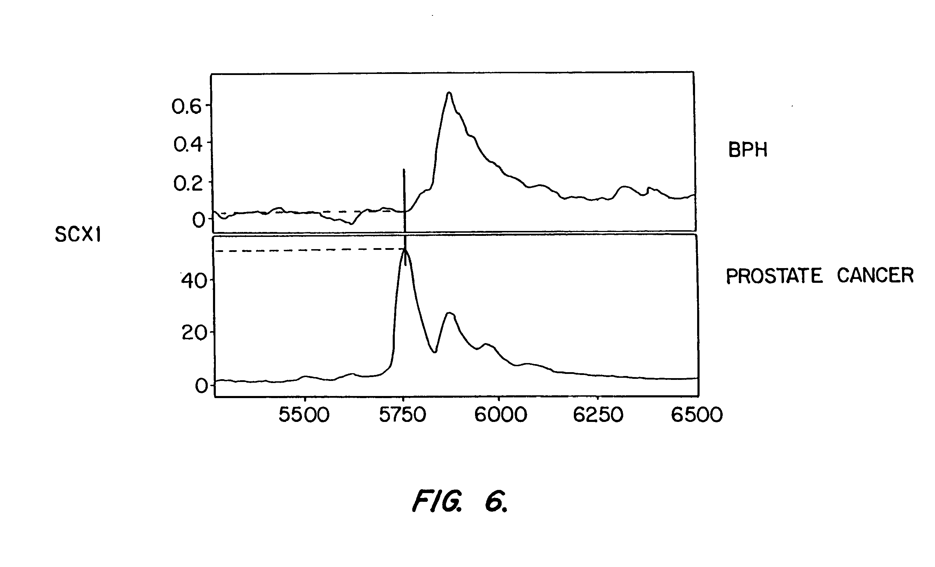

The results are shown in Figure 6. The top panel shows the proteins from the sample of the BPH patient that are desorbed from the SCX1 ProteinChip^{®} in the spectral view format. As shown in Figure 6, only a negligible amount (relative intensity of less than 0.1) of desorbed protein was observed at an apparent molecular weight of about 5753 Da. The bottom panel shows the proteins from the sample of the prostate cancer patient that are desorbed from the SCX1 ProteinChip^{®} in the spectral view format. As shown in Figure 6, a protein having apparent molecular weight of about 5753 Da was present at a high level (relative intensity of about 52) in the sample of the prostate cancer patient.

This protein having an apparent molecular weight of 5753 Da was subsequently peptide mapped with two proteolytic enzymes, trypsin and V8 protease. By matching with proteins in the Swiss Prot Database, the marker was identified to be seminal basic protein, which is a proteolytic fragment generated by PSA-mediated proteolysis of semenogelin I. *See,* Figure 1 for the amino acid sequence of seminal basic protein.

The present invention provides methods for aiding prostate cancer diagnosis using markers that are differentially present in samples of a prostate cancer patient and a subject who does not have prostate cancer (*e.g.,* a benign prostate hyperplasia patient). While specific examples have been provided, the above description is illustrative and not restrictive.

## Claims

1. A method for aiding a prostate cancer diagnosis, the method comprising:
i. determining by mass spectroscopy a test amount of a plurality of protein markers in a sample from a subject, the protein markers having apparent molecular weights of less than 10,000 Da;
ii. comparing the test amount of the plurality of protein markers having apparent molecular weight of less than 10,000 Da with an amount of a plurality of protein markers having an apparent molecular weight of less than 10,000 Da from a control sample where the control sample originates from benign prostate hyperplasia; and
iii. determining whether the test amount is a diagnostic amount consistent with a diagnosis of prostate cancer versus benign prostate hyperplasia.

2. The method of claim 1, wherein the sample is selected from the group consisting of blood, serum, urine, semen, seminal fluid, seminal plasma, and tissue extracts.

3. The method of claim 1, the method further comprising:
(a) generating data on the sample with the mass spectrometer indicating intensity of signal for mass/charge ratios;
(b) transforming the data into computer-readable form; and
(c) operating a computer to execute an algorithm, wherein the algorithm determines closeness-of-fit between the computer-readable data and data indicating a diagnosis of prostate cancer or a negative diagnosis.

4. The method of claim 1, wherein the sample is seminal plasma.

5. The method of claim 1 where the protein markers are adsorbed onto a probe comprising an adsorbent of a hydrophilic polymer.

6. The method of claim 1 where the protein markers are adsorbed onto a probe comprising a metal binding group.

7. The method of claim 5 where the adsorbent comprises a hydrophobic group.

8. The method of claim 5 where the adsorbent comprises a cationic group.

9. The method of claim 5 where the adsorbent comprises a metal ion chelating group.

## Patentansprüche

1. Verfahren zur Unterstützung einer Prostatakrebsdiagnose, wobei das Verfahren umfasst:
(i) Bestimmen einer Testmenge einer Vielzahl von Proteinmarkem in einer Probe aus einem Patienten durch Massenspektroskopie, wobei die Proteinmarker scheinbare Molekulargewichte von weniger als 10.000 Da aufweisen;
(ii) Vergleichen der Testmenge der Vielzahl von Proteinmarkem mit einem scheinbaren Molekulargewicht von weniger als 10.000 Da mit einer Menge einer Vielzahl von Proteinmarkem mit einem scheinbaren Molekulargewicht von weniger als 10.000 Da aus einer Kontrollprobe, wobei die Kontrollprobe aus einer gutartigen Prostatahyperplasie stammt; und
(iii) Bestimmen, ob die Testmenge eine diagnostische Menge ist, die einer Diagnose von Prostatakrebs gegenüber einer gutartigen Prostatahyperplasie entspricht.

2. Verfahren nach Anspruch 1, bei dem die Probe ausgewählt wird aus der Gruppe, bestehend aus Blut, Serum, Urin, Samen, Samenflüssigkeit, Samenplasma und Gewebeextrakten.

3. Verfahren nach Anspruch 1, wobei das Verfahren außerdem umfasst:
(a) Erzeugen von Daten auf der Probe mit dem Massenspektrometer, die die Intensität des Signals für Massen/Ladungs-Verhältnisse anzeigen;
(b) Umwandeln der Daten in eine computerlesbare Form; und
(c) Einsetzen eines Computers, um einen Algorithmus durchzuführen, wobei der Algorithmus die Passgenauigheit zwischen den computerlesbaren Daten und den die Diagnose eines Prostatakrebses oder eine negative Diagnose anzeigenden Daten bestimmt.

4. Verfahren nach Anspruch 1, bei dem die Probe Samenplasma ist.

5. Verfahren nach Anspruch 1, bei dem die Proteinmarker auf eine Sonde adsorbiert werden, die ein Adsorptionsmittel aus einem hydrophilen Polymer umfasst.

6. Verfahren nach Anspruch 1, bei dem die Proteinmarker auf eine Sonde adsorbiert werden, die eine metallbindende Gruppe umfasst.

7. Verfahren nach Anspruch 5, bei dem das Adsorptionsmittel eine hydrophobe Gruppe umfasst.

8. Verfahren nach Anspruch 5, bei dem das Adsorptionsmittel eine kationische Gruppe umfasst.

9. Verfahren nach Anspruch 5, bei dem das Adsorptionsmittel eine Metallionchelatbildende Gruppe umfasst.

## Revendications

1. Procédé pour contribuer au diagnostic du cancer de la prostate, le procédé comprenant :
i. la détermination par spectrométrie de masse d'une quantité de test d'une pluralité de marqueurs protéiques dans un échantillon d'un sujet, les marqueurs protéiques ayant des masses moléculaires apparentes inférieures à 10 000 Da ;
ii. la comparaison de la quantité de test de la pluralité de marqueurs protéiques ayant une masse moléculaire apparente inférieure à 10 000 Da à une quantité d'une pluralité de marqueurs protéiques ayant une masse moléculaire apparente inférieure à 10 000 Da d'un échantillon de contrôle où l'échantillon de contrôle provient d'une hyperplasie bénigne de la prostate ; et
iii. la détermination du fait que la quantité de test est ou non une quantité de diagnostic cohérente avec un diagnostic du cancer de la prostate versus l'hyperplasie bénigne de la prostate.

2. Procédé selon la revendication 1, dans lequel l'échantillon est choisi dans le groupe constitué par le sang, le sérum, l'urine, le sperme, le liquide séminal, le plasma séminal et des extraits de tissu.

3. Procédé selon la revendication 1, le procédé comprenant en outre :
(a) la génération de données relatives à l'échantillon avec le spectromètre de masse indiquant l'intensité de signal pour les rapports masse/charge ;
(b) la transformation des données en une forme pouvant être lue par un ordinateur ; et
(c) l'utilisation d'un ordinateur pour exécuter un algorithme, dans lequel l'algorithme détermine la qualité de l'ajustement entre les données pouvant être lues par un ordinateur et les données indiquant un diagnostic du cancer de la prostate ou un diagnostic négatif.

4. Procédé selon la revendication 1, dans lequel l'échantillon est du plasma séminal.

5. Procédé selon la revendication 1, dans lequel les marqueurs protéiques sont adsorbés sur une sonde comprenant un adsorbant d'un polymère hydrophile.

6. Procédé selon la revendication 1, dans lequel les marqueurs protéiques sont adsorbés sur une sonde comprenant un groupe de liaison au métal.

7. Procédé selon la revendication 5, dans lequel l'adsorbant comprend un groupe hydrophobe.

8. Procédé selon la revendication 5, dans lequel l'adsorbant comprend un groupe cationique.

9. Procédé selon la revendication 5, dans lequel l'adsorbant comprend un groupe chélateur d'ions métalliques.
